(19) **Europäisches Patentamt European Patent Office Office européen des brevets**

(11) **EP 1 557 180 B1**

(12) **EUROPEAN PATENT SPECIFICATION**

(45) Date of publication and mention of the grant of the patent:
**30.06.2010 Bulletin 2010/26**

(51) Int Cl.:
*A61L 2/18* (2006.01)     *A61L 12/12* (2006.01)
*A61L 12/14* (2006.01)

(21) Application number: **03809428.0**

(22) Date of filing: **08.09.2003**

(86) International application number:
**PCT/JP2003/011416**

(87) International publication number:
**WO 2004/037302 (06.05.2004 Gazette 2004/19)**

(54) **DISINFECTION METHOD**

DESINFEKTIONSMETHODE

METHODE DE DESINFECTION

(84) Designated Contracting States:
**DE FR GB**

(30) Priority: **28.10.2002 JP 2002312524**

(43) Date of publication of application:
**27.07.2005 Bulletin 2005/30**

(73) Proprietor: **Menicon Co., Ltd.**
**Nagoya-shi, Aichi 460-0006 (JP)**

(72) Inventors:
• **SAKANISHI, Kotaro,**
**c/o Menicon Co., Ltd.**
**Kasugai-shi,**
**Aichi-ken (JP)**

• **NAKADA, Kazuhiko,**
**c/o Menicon Co., Ltd.**
**Kasugai-shi,**
**Aichi-ken (JP)**

(74) Representative: **Paget, Hugh Charles Edward et al**
**Mewburn Ellis LLP**
**33 Gutter Lane**
**London**
**EC2V 8AS (GB)**

(56) References cited:
EP-A- 0 766 970       EP-A2- 1 095 664
EP-A2- 1 095 664     WO-A-00/19981
WO-A-92/11042       WO-A-03/015834
WO-A1-00/19981     WO-A1-92/11042
WO-A1-03/015834   US-A- 5 660 862
US-A- 6 165 415

**Description**

The present invention relates in general to a disinfection method, and more particularly to such a method for disinfecting, in particular, medical instruments such as contact lenses and contact mirrors for an ophthalmic use, by using a disinfecting liquid which has an excellent disinfecting effect.

BACKGROUND ART

Conventionally, contact lenses are classified into non-water-contained contact lenses and water-contained contact lenses, or hard contact lenses and soft contact lenses. During a long period of use of the contact lenses, microorganisms such as bacteria and fungi tend to adhere to and proliferate on the surface of the contact lenses while the contact lenses are stored after they have been removed from the eyes. Such microorganisms may cause infectious diseases, giving adverse influences on the eyes of the user. In view of the above, the contact lenses generally need to be disinfected before they are worn on the eyes. In particular, it is indispensable to disinfect the soft contact lenses, since the microorganisms are likely to proliferate on the surface of the soft contact lenses more often than on the hard contact lenses, increasing an anxiety of causing the infectious diseases.

For disinfecting the contact lenses, there have been principally practiced a thermal disinfection method using a suitable boiling and disinfecting device, and a chemical disinfection method using a suitable disinfectant or a preservative. The thermal disinfection method undesirably requires a time-consuming boiling operation to disinfect the contact lenses. Accordingly, in recent years, the chemical disinfection method has been widely employed to disinfect the contact lenses.

In the chemical disinfection method, a liquid agent for contact lenses which contains a suitable disinfectant/ preservative is used. The contact lenses are immersed in the liquid agent, so that the contact lenses are disinfected. As the disinfectant/preservative contained in the liquid agent, various chemical agents have been proposed. For instance, chlorhexidine, benzalkonium chloride, and thimerosal are known. (See JP-A-52-109953, JP-A-62-153217, and JP-A-63-59960, etc.).

When the contact lenses are disinfected by the chemical disinfection method, the disinfectant/preservative such as chlorhexidine described above is contained generally in a relatively high concentration in the contact lens liquid agent, for the purpose of obtaining a sufficiently high disinfecting effect. In this case, the disinfectant/preservative such as chlorhexidine is likely to be adsorbed on the contact lenses on a molecular level, deteriorating the wettability at the surface of the contact lenses, and causing a change in the physical properties and the configuration of the contact lenses. In some cases, the adsorption of the disinfectant/ preservative on the contact lenses may cause various troubles with the eyes such as an allergy while the contact lenses are worn on the eyes. To keep the physical properties and the configuration of the contact lenses unchanged, and assure a high degree of safety with the eyes of the lens users, the contact lenses may be disinfected by using a liquid agent in which the disinfectant/preservative is contained in a relatively low concentration. In this case, however, the disinfecting effect to be exhibited by the liquid agent is inevitably lowered, causing contamination of the contact lenses by the microorganisms. Accordingly, when the contact lenses are disinfected by using such a conventional liquid agent, it is necessary to exercise an utmost care in adjusting the concentration of the disinfectant/preservative included in the liquid agent, making the disinfecting treatment of the contact lenses very cumbersome.

As the disinfectant/preservative used in the chemical disinfection method, hydrogen peroxide is also well known. When the hydrogen peroxide is used as the disinfectant/preservative, however, the concentration of the hydrogen peroxide needs to be generally as high as 3% (30,000 ppm) to obtain an intended disinfecting effect. In addition, it is necessary to carry out a neutralization treatment, after the disinfection of the contact lenses, for neutralizing the hydrogen peroxide remaining on the surface of the contact lenses or in the contact lenses, to decompose and detoxify the hydrogen peroxide. The residual hydrogen peroxide remaining even in a small amount may cause an anxiety of giving serious troubles to the eyes of the user. Therefore, the neutralization treatment has been conventionally conducted for completely decomposing and removing the residual hydrogen peroxide, by rinsing the disinfected contact lenses with a physiological salt solution, or by using a metal catalyst such as platinum black, a reducing agent such as sodium sulfite, sodium thiosulfate, or pyruvic acid, or an enzyme catalyst such as catalase or peroxidase. Thus, the chemical disinfection method using the hydrogen peroxide requires the neutralization treatment for neutralizing the residual hydrogen peroxide remaining after the disinfecting treatment, undesirably making the disinfecting treatment very cumbersome.

For this reason, the applicant of the present application have made various studies to solve the above-mentioned problem, and found that disinfecting effect of hydrogen peroxide is improved by simply irradiating a disinfecting liquid including hydrogen peroxide, in which an object to be disinfected, such as a contact lens, is immersed with a light having a predetermined wavelength, eliminating a neutralization treatment for neutralizing the hydrogen peroxide after the disinfection. Based on the findings, the applicant of the present application filed another application (JP-A-2003-135572). However, the inventors of the present invention have made a further study on the proposed disinfection method, and have found a method, which is different from the above-described method. The inventors of the present invention have also found that the different method also exhibits an excellent disinfecting effect.

Irradiation of items immersed in a liquid containing low amounts of hydrogen peroxide in conjunction with a metal ion EDTA complex is known from EP-A-1 426 064. A similar method is disclosed in EP-A-1 095 664.

DISCLOSURE OF THE INVENTION

The present invention was developed in the light of the background art situations described above. It is an object of the present invention to provide a disinfection method which can be conducted in a simplified manner and which assures an excellent disinfecting effect.

The present invention was made to solve the problem described above. The present invention provides a method of disinfecting an object by being immersed in a disinfecting liquid as defined in claim 1.

In the disinfection method according to the present invention, the object to be disinfected is immersed in the disinfecting liquid, which includes, in the aqueous medium, the hydrogen peroxide and the metal-EDTA (ethylenediamine tetraacetic acid) complex, wherein the concentration of the hydrogen peroxide is 1000 ppm or less in the disinfecting liquid. According to the present method, although the concentration of the hydrogen peroxide is as low as 1000 ppm or less, there are formed hydroxyradical ($\cdot$OH) having a strongly-oxidative action or power. Owing to the strongly-oxidative power of the hydroxyradical, the disinfecting activity to kill harmful microorganisms such as bacteria is remarkably improved by the metal-EDTA complex, and very high sterilizing effects (disinfecting effects) are advantageously obtained.

The hydrogen peroxide included in the disinfecting liquid is contained in the disinfecting liquid in a considerably low concentration, so that the most of the hydrogen peroxide in the disinfecting liquid is effectively decomposed. Therefore, it is not necessary to carry out the neutralization treatment for neutralizing the remaining hydrogen peroxide after the disinfecting treatment.

In one preferred form of the disinfection method according to the present invention, the concentration of the hydrogen peroxide is in a range of 10-500 ppm in the disinfecting liquid. The present invention advantageously assures an excellent disinfecting efficacy even if the concentration of the hydrogen peroxide is relatively low.

In another preferred form of the present invention, the concentration of the metal-EDTA complex is generally in a range of 10-1200 ppm in the disinfecting liquid, and the metal-EDTA complex is an EDTA Fe $\cdot$ Na.

In the disinfection method according to the present invention, the disinfecting liquid, in which the object to be disinfected is immersed, is irradiated with a visible light. According to this arrangement, the irradiated hydrogen peroxide included in the disinfecting liquid is advantageously decomposed in a short period of time, for thereby assuring a high degree of disinfecting effect, as well as decomposing the hydrogen peroxide which is harmful to a living body. Accordingly, the present arrangement can enjoy the advantages of eliminating the neutralization treatment for neutralizing the hydrogen peroxide after the disinfecting treatment.

In the present method, a visible light is employed as the light for the irradiation, so that it is possible to reduce the weight of a light emitting device and necessary power, compared with an irradiation of another light, e.g., a UV light. Moreover, the present method can avoid giving adverse influences on the retina of the user's eye, which may be caused because of a malfunction or a misuse of the light emitting device while the disinfection is being carried out.

In another preferred form of the disinfection method according to the present invention, in the irradiation of the disinfecting liquid with the visible light, the disinfecting liquid is accommodated in a suitable container such that the disinfecting liquid in the container has a depth or a thickness of 3-10 mm, and then the object to be disinfected is immersed, and the disinfecting liquid is sufficiently irradiated with the light at least in the direction of the depth or the thickness of the disinfecting liquid accommodated in the container. In irradiating the disinfecting liquid with the light as described above, the disinfecting liquid needs to be accommodated in a suitable container such that the disinfecting liquid in the container has a depth or a thickness as small as 3-10 mm, so that the hydrogen peroxide included in the disinfecting liquid can be uniformly and sufficiently irradiated with the light, and thereby the hydrogen peroxide is still more effectively decomposed.

As the above-mentioned visible light, it is preferable to use a light having a wavelength of 390-600 nm. Therefore, as mentioned above, the present invention can reduce the weight of the light emitting device and necessary power, while it assures a high degree of disinfecting effect.

In the disinfecting liquid used in the present disinfection method, the osmotic pressure is held preferably in a range of 250-350 mOsm. This arrangement assures a significantly high degree of safety with the living body such as eyes, as well as this arrangement prevents the object such as contact lenses from being adversely influenced.

In the disinfection method according to the present invention, the disinfecting liquid may further include, as needed, a surfactant, so that a cleaning effect owing to the surfactant can be added to the disinfecting liquid.

In still another preferred form of the disinfection method according to the present invention, the object to be disinfected includes, for example, the medical instruments such as contact lenses and contact mirrors for an ophthalmic use. In particular, the contact lenses are advantageously disinfected according to the present method.

BEST MODE FOR CARRYING OUT THE INVENTION

The above-indicated disinfection method may be attained according to the present invention according to claim 1, in which the object to be disinfected, such as the medical instruments including the contact lenses and the ophthalmic contact mirrors, are immersed in the disinfecting liquid, in which the hydrogen peroxide and the metal-EDTA complex is included in an aqueous medium such as purified water, so that the concentration of the hydrogen peroxide is in a range of 1000 ppm or less in the disinfecting liquid. In particular, the present invention is **characterized in that** it employs the disinfecting liquid which includes the hydrogen peroxide, which has a low concentration in the disinfecting liquid, in combination with the metal ion or the metal-EDTA complex.

More specifically described, the hydrogen peroxide ($H_2O_2$), which is included and dissolved in the disinfecting liquid of the present invention, is conventionally used as the disinfectant, and upon decomposition of the hydrogen peroxide, there are formed hydroxyradical (•OH) having a strong oxidative action or power. Owing to the strong oxidative power of the hydroxyradical, the harmful microorganisms are effectively killed. To obtain the desired disinfecting effect, generally the hydrogen peroxide needs to be used in a concentration of about 3% (30,000 ppm) in the disinfecting liquid. However, due to the combination with the metal-EDTA complex, the concentration of the hydrogen peroxide in the disinfecting liquid of the present invention can be as low as 1000 ppm or less, and more preferably as low as 10-500 ppm.

If the concentration of the hydrogen peroxide in the disinfecting liquid is more than 1000 ppm, it still assures a sufficient disinfecting effect, which is similar to that of the conventionally used disinfecting liquid. However, there may be a problem that some of the hydrogen peroxide to be not decomposed remains in the disinfecting liquid. If a contact lens is employed as the object to be disinfected, some hydrogen peroxide remains on or inside the contact lens after the disinfection, and this would give an adverse influence on the user's eye, such as a trouble to the user's eye. In this case, the conventional neutralization treatment needs to be conducted for neutralizing the hydrogen peroxide remaining in the disinfecting liquid, for thereby making the process of the disinfection cumbersome.

On the other hand, the metal-EDTA complex is added to the disinfecting liquid together with the above-mentioned hydrogen peroxide to promote the decomposition of the hydrogen peroxide so as to effectively generate the hydroxyradical. Therefore, the metal-EDTA complex to be added to the disinfecting liquid is not particularly limited, but the metal ion for the complex may be suitably selected from among an iron ion, a nickel ion, and a copper ion, for instance, which can cause a Fenton reaction which is one of the reactions of decomposition of the hydrogen peroxide. The metal-EDTA complex may be suitably selected from among EDTA complexes of the above-mentioned metal ions, for instance, an Fe-EDTA complex, an Ni-EDTA complex, or a Cu-EDTA complex. The metal-EDTA complex added to the disinfecting liquid which includes the hydrogen peroxide effectively promotes the decomposition of the hydrogen peroxide, and increases the concentration of the hydroxyradical in the disinfecting liquid, for thereby assuring the disinfecting efficacy of the disinfecting liquid, even if the concentration of the hydrogen peroxide is 1000 ppm or less.

According to the procedure described above, the combination of the hydrogen peroxide and the metal-EDTA complex enhances the disinfecting efficacy of the disinfecting liquid, so that a highly efficient disinfecting effect can be provided. Therefore, the present invention can lower the concentration of the hydrogen peroxide which would give an adverse influence to the living body, and can assure the disinfecting efficacy of the disinfecting liquid, even if the concentration of the hydrogen peroxide is 1000 ppm or less. In particular, if the concentration of the hydrogen peroxide as low as about 10-500 ppm is employed, most of the hydrogen peroxide contained in the disinfecting liquid can be decomposed, leaving very little amount of residual hydrogen peroxide in the disinfecting liquid. Therefore, the present arrangement does not need conventionally required neutralization treatment for neutralizing the remaining hydrogen peroxide in the disinfecting liquid, so that the present arrangement enjoys the advantages of high economy and easy handling of the disinfecting liquid.

The concentration of the metal-EDTA complex to promote the decomposition of the hydrogen peroxide is preferably 10-1200 ppm, and more preferably 100-1000 ppm in the disinfecting liquid. This is because if the concentration of the metal-EDTA complex is less than 10 ppm, the effect provided by the addition of the metal-EDTA complex cannot be sufficiently assured. On the other hand, if the concentration is more than 1200 ppm, it does not improve the disinfecting efficacy of the disinfecting liquid, and it lowers the economy.

The disinfecting liquid which includes a suitable amounts of the hydrogen peroxide and the metal-EDTA complex described above may further include a surfactant, so that the disinfecting liquid has an activity for cleaning the object to be disinfected, in addition to the disinfecting activity for killing the microorganisms. The surfactant to be added to the present disinfecting liquid may be suitably selected from among known anionic, nonionic, amphoteric, and cationic surfactants, which are generally used in the conventional contact lens liquid agents. Owing to the addition of the surfactant, the disinfecting liquid exhibits a cleaning effect such as removal of the lipid, etc., from the object to be disinfected.

In addition to the above, the present disinfecting liquid may further include, as needed, various known additives as usually used in the conventional disinfecting liquid. For instance, the present disinfecting liquid may further contain any combination of a chelating agent, a buffer, a thickener, a preservative, a disinfectant, an oxidizing agent, and an isotonic agent. The additives to be included in the disinfecting liquid should be safe to the living body and it should not give an adverse influence on the effects provided by the hydrogen peroxide. The additives are included in the disinfecting liquid in amounts that do not inhibit the above-mentioned disinfecting effect.

The chelating agent to be added, if necessary, as one of the additives to the disinfecting liquid is generally effective to prevent metal ions, such as calcium, from adhering to the object to be disinfected. Examples of the chelating agent include ethylenediamine tetraacetic acid, citric acid, gluconic acid, tartaric acid, phytic acid and salts thereof (e.g., sodium salt). The buffer is included in the disinfecting liquid in order to adjust the pH of the disinfecting liquid to an appropriate level, for the purpose of maintaining the physical properties and the configuration of the object to be disinfected. Typical examples of the buffer are acids, such as citric acid, malic acid, lactic acid, ascorbic acid, maleic acid, gluconic acid, phosphoric acid, boric acid, amino acid such as glycine and glutamic acid, tris(hydroxymethyl)amino methane, and salts of those acids (e.g., sodium salt).

As the thickener used to adjust the viscosity of the disinfecting liquid, a suitable viscous base, such as polyvinyl alcohol, poly-N-vinylpyrrolidone, polyethylene glycol, polyacrylamide, a hydrolyzed product of polyacrylamide, polyacrylic acid, hydroxyethyl cellulose, carboxymethyl cellulose, methylhydroxyethyl cellulose, methylhydroxypropyl cellulose, methyl cellulose, gelatin, sodium alginate, sodium chondroitin sulfate, xanthan gum, gum Arabic, or guar gum may be named. The disinfecting liquid may include, as the preservative/disinfectant or the oxidizing agent, various known compounds, which exhibit the preservative or disinfecting efficacy, in addition to the hydrogen peroxide employed in the present invention. Examples of the oxidizing agent include peroxides such as ozone water, sodium peroxide, magnesium peroxide, and silver oxide. Owing to the addition of the oxidizing agent (peroxide), the disinfecting liquid exhibits a further improved disinfecting effect.

The present disinfecting liquid preferably has an osmotic pressure of 250-350 mOsm, for protecting the living body from being adversely influenced when the disinfected object is used with the disinfecting liquid remaining on and adhering to its surface. To adjust the osmotic pressure of the disinfecting liquid to a desired level, a suitable amount of various known isotonic agents such as sodium chloride or potassium chloride is preferably added to the disinfecting liquid. Where the sodium chloride is used as the isotonic agent, the amount of the sodium chloride to be added is preferably in a range of 0.7-1.2 wt.%, and more preferably in a range of 0.8-1.1 wt.%, based on the total weight of the disinfecting liquid. Where the contact lens is disinfected by using the present disinfecting liquid, the irritation to the eye of the user is advantageously reduced, even if the contact lens is worn on the eye with the disinfecting liquid adhering to its surface and the disinfecting liquid gets in the eye, since the osmotic pressure of the disinfecting liquid is substantially equal to that of the tear fluid.

As the aqueous medium, in which various components described above are included, there may be employed a solution which is principally constituted by water, in addition to tap water, purified water, and distilled water. Such a solution includes a saline solution, and a known aqueous liquid agent such as a contact lens storing liquid or a cleaning liquid.

In the present invention, the object to be disinfected, such as a contact lens, is immersed in the disinfecting liquid which includes the various components described above, so that the object is disinfected.

In the present invention, the disinfecting liquid includes the metal-EDTA complex together with the hydrogen peroxide, for thereby the hydrogen peroxide is effectively decomposed, unlike the conventional disinfecting liquid in which the hydrogen peroxide is gradually decomposed. Therefore, the concentration of the hydroxyradical formed upon decomposition of the hydrogen peroxide is increased within a relatively short period of time, so that the harmful microorganisms present in the disinfecting liquid can be effectively killed owing to the strong oxidative power of the hydroxyradical. Accordingly, the object immersed in the disinfecting liquid is sufficiently disinfected owing to the excellent disinfecting efficacy.

As described above, the present invention assures a sufficiently high disinfecting effect by simply immersing the object to be disinfected in the disinfecting liquid. If the disinfecting liquid in which the object to be disinfected is immersed is irradiated with the light, the irradiated hydrogen peroxide is effectively decomposed in a short period of time, for thereby a further enhanced disinfecting effect can be obtained.

In irradiating the disinfecting liquid, the disinfecting liquid is generally accommodated in a predetermined container such that the disinfecting liquid accommodated in the container has a depth or a thickness of 3-10 mm, and the object to be disinfected is immersed in the disinfecting liquid. In this state, the hydrogen peroxide in the disinfecting liquid is uniformly and sufficiently irradiated with the light, so that the hydrogen peroxide, which has been exposed to the light, is effectively decomposed. Accordingly, the disinfecting efficacy can be further improved. If the depth (thickness) of the disinfecting liquid in the container is less than 3 mm, the amount of the disinfecting liquid is undesirably small, and the amount of the hydrogen peroxide included in the disinfecting liquid is accordingly small, resulting in an insufficient disinfecting effect. In this case, depending on the object to be disinfected, the object may not be entirely immersed in the disinfecting liquid, causing a problem that a local portion of the object, which is not immersed in the disinfecting liquid, is not disinfected. On the other hand, if the depth (thickness) of the disinfecting liquid in the container exceeds 10 mm, it causes a problem that the light is not sufficiently incident on the hydrogen peroxide existing in the disinfecting liquid.

The above-mentioned predetermined container for the irradiation is not particularly limited, but may be suitably selected from among any known various containers, as long as the containers have sizes sufficient for accommodating the object to be disinfected, and permit the disinfecting liquid to have a depth or a thickness of 3-10 mm when accommodated in the containers, and as long as the containers permit the incident light to transmit therethrough in at least the direction of the depth or the thickness of the disinfected liquid accommodated therein. The container is preferably formed of a light-transmitting material, so that the light is incident on the disinfecting liquid in a plurality of directions. If the disinfecting liquid accommodated in the thus formed container is irradiated with the light in an additional direction other than the direction of its depth or thickness, in addition to the direction of its depth or thickness, the efficiency of the light irradiation effectively increased, resulting in a further improved disinfecting effect.

The light to be incident on the disinfecting liquid in which the object is immersed is a visible light, in particular, a visible light, which has a wavelength in a range of 390-600 nm. As a light source to irradiate the visible light, a light-emitting diode (LED), is used according to the invention, and this selection contributes to reduce the weight of the light emitting device and necessary power, compared with the use of other lights, such as a UV light.

The intensity of the light is not particularly limited. However, if the intensity of the light is excessively low, the hydrogen peroxide is not sufficiently decomposed, so that the intended disinfecting effect cannot be attained. On the contrary, if the intensity of the light is excessively high, this may give an adverse influence on the object, which is immersed in the disinfecting liquid. For instance, the contact lens may suffer from deterioration such as a change of its color to yellow, or a reduction in its UV-absorbing power. Therefore, it is preferable to select a suitable intensity based on the light source to be used.

The period of time during which the object is disinfected by the disinfecting liquid is suitably determined depending upon a desired degree of disinfection of the object, by taking account of various factors such as the kind of the object to be disinfected. For obtaining the desired disinfecting effect to a sufficient extent without the irradiation of the light, it is generally needed to immerge the object in the disinfecting liquid for not less than 10 minutes. If the disinfecting liquid is irradiated with the light, it is generally needed to immerge the object in the disinfecting liquid or to irradiate the disinfecting liquid for not less than 5 minutes. The period of time during which the object is disinfected by the disinfecting liquid is up to about 12 hours, preferably up to about 6 hours, to improve the efficiency of the disinfecting operation, regardless of the performance of the light irradiation.

According to the present method as described above, the object can be easily and effectively disinfected without making the handling of the disinfecting liquid difficult.

The object, which has been disinfected according to the present method, is usually used after it is taken out of the disinfecting liquid. When the disinfecting liquid includes the hydrogen peroxide in a concentration held in the above-described range (10-500 ppm), the amount of the hydrogen peroxide remaining in the disinfecting liquid without being decomposed is extremely small, i.e., almost zero. Accordingly, it is not necessary to conduct the neutralization treatment to neutralize the hydrogen peroxide. The object may be used without any subsequent treatment, or may be rinsed with a saline, for instance, before the object is used.

The object to be disinfected by the disinfecting liquid according to the present invention includes the medical instruments, particularly contact lenses and contact mirrors for the ophthalmic use. In particular, the contact lenses can be considerably effectively disinfected according to the present invention. The kinds of the contact lenses to be disinfected according to the present invention are not particularly limited. For instance, the present invention can be applied to low-water-content and high-water-content soft contact lenses, and hard contact lenses, irrespective of the materials of those contact lenses.

EXAMPLE

To further clarify the concept of the present invention, some examples of the invention will be described.

<Preparation of disinfecting liquid specimens>

Initially, various disinfecting liquid specimens Nos. 1-12 were prepared in the following manner. The hydrogen peroxide ($H_2O_2$), the metal ion, and the metal-EDTA complex were mixed in respective concentrations indicated in the following TABLE 1, with a separately-prepared sterile saline solution (aqueous NaCl solution) containing sodium chloride (NaCl) in a concentration of 0.9 wt.%. The metal ion was introduced into the disinfecting liquid specimen by dissolving iron (III) chloride while the metal-EDTA complex was introduced in the disinfecting liquid specimen by dissolving EDTA Fe • Na complex. It was confirmed that each of the above specimens Nos. 1-12 had osmotic pressure value in a range of about 280-290 mOsm.

<Test for examining the disinfecting effect>

Each of the disinfecting liquid specimens, which were prepared as mentioned above, was examined of its disinfecting effect in the following manner. Initially, the disinfecting liquid specimens were put into respective sterile plates each having an inside diameter of 9 cm, such that the disinfecting liquid specimens accommodated in the respective plates had a depth of about 8 mm. Then, *S.a.* (*Serratia marcescens: ATCC 13880*) as the test bacterium was inoculated into the respective disinfecting liquid specimens at about $10^6$ cfu/mL. Each of the disinfecting liquid specimen Nos. 3, 4, 6, 9, 10, and 12 was irradiated with a visible light for one hour, using a blue light-emitting diode (wavelength: 470 nm) or a fluorescent lamp, such that the light was incident on the upper side of the plate in a direction of the depth of the disinfecting liquid specimen accommodated in the plate. The disinfecting liquid specimen Nos. 1, 2, 5, 7, 8, and 11 were kept in a dark place for one hour.

Viable microorganism count of each of the disinfecting liquid specimens was calculated. A predetermined amount of the disinfecting liquid specimens was taken out of the respective plates, and diluted with a sterile saline solution, so as to provide diluted samples. The viable microorganism counts per 1 mL of the thus obtained diluted samples were respectively measured according to a plate method. On the basis of the measured viable microorganism counts, there were calculated viable microorganism counts per 1 mL of the each of the disinfecting liquid specimens after irradiation with the light. Then, an amount of reduction of the bacteria was calculated in logarithm (log reduction) according to the following equation. The results of calculation are indicated in the following TABLE 1,

$$\text{Bacteria reduction amount [in logarithm]} = \\ \log \text{(viable microorganism count per 1 mL of each specimen immediately after inoculation)} - \\ \log \text{(viable microorganism count per 1 mL of each specimen after irradiation with the light)}$$

## TABLE 1

| | | Concentration [ppm] | | | Irradiation of the light (kind of the light source) | Log reduction |
|---|---|---|---|---|---|---|
| Specimen No. | | H₂O₂ | Metal -EDTA complex | Metal ion | | |
| | 1 | 30 | 1000 | – | No (dark place) | 2.9 |
| | 2 | 300 | 100 | – | No (dark place) | 2.7 |
| | 3 | 300 | 100 | – | Yes (blue LED) | 5.2 |
| | 4 | 300 | 100 | – | Yes (fluorescent lamp) | 2.9 |
| | 5 | 300 | 1000 | – | No (dark place) | 3.2 |
| | 6 | 300 | – | 50 | Yes (fluorescent lamp) | 3.0 |
| | 7 | 30 | – | – | No (dark place) | 0.4 |
| | 8 | 300 | – | – | No (dark place) | 1.1 |
| | 9 | 300 | – | – | Yes (blue LED) | 2.2 |
| | 10 | 300 | – | – | Yes (fluorescent lamp) | 1.2 |
| | 11 | – | – | – | No (dark place) | ·0.4 |
| | 12 | – | – | – | Yes (blue LED) | -0.2 |

As it is apparent from the results indicated in the above TABLE 1, in each of the disinfecting liquid specimens Nos. 1-6 which includes the metal ion or the metal-EDTA complex together with the hydrogen peroxide, the log reduction value indicative of the amount of reduction of the bacteria was not less than 2.7. Accordingly, it is recognized that a high degree of disinfecting effect can be obtained by immersing the object in such disinfecting liquids. In particular, the specimen No. 3, which was irradiated with the visible light of LED, exhibited especially high degree of disinfecting effect.

The disinfecting liquid specimen Nos. 7-12, on the contrary, did not exhibit a sufficient disinfecting effect compared with the specimen Nos. 1-6, since the specimen Nos. 7-12 do not include either of the Fe ion and the Fe-EDTA complex, and some of the specimen Nos. 7-12 further lack the hydrogen peroxide.

INDUSTRIAL APPLICABILITY

As it is apparent from the foregoing description, the disinfection method according to the present invention uses the disinfecting liquid in which the hydrogen peroxide is contained in combination with the metal-EDTA complex. In the present method, the object to be disinfected is immersed in the disinfecting liquid, so that the disinfecting efficacy of the hydrogen peroxide is effectively increased and an excellent disinfecting effect is assured. The present method further assures an improved disinfecting effect by irradiating, with the visible light, the disinfecting liquid in which the object to be disinfected is immersed. According to the present method, the hydrogen peroxide can be effectively decomposed by irradiating with the light, so that the concentration of the hydrogen peroxide included in the disinfecting liquid can be made much lower than that in the conventional disinfecting liquid. In addition, the present method does not necessarily require the neutralization treatment for neutralizing the residual hydrogen peroxide after the disinfecting treatment.

These effects are also assured for objects to be disinfected, which are other than medical instruments such as contact lenses and contact mirrors for an ophthalmic use.

**Claims**

1.   A method of disinfecting an object by being immersed in a disinfecting liquid, **characterized in that:**

said disinfecting liquid contains, in an aqueous medium, a hydrogen peroxide and a metal-EDTA complex, wherein a concentration of said hydrogen peroxide is in a range of 1000 ppm or less in the disinfecting liquid, wherein said disinfecting liquid, in which said object is immersed, is further irradiated with a visible light, form a light-emitting diode.

2.   A method according to claim 1, wherein a concentration of said hydrogen peroxide is in a range of 10-500 ppm in said disinfecting liquid.

3.   A method according to claim 1 or 2, wherein a concentration of said metal-EDTA complex is in a range of 10-1200 ppm in said disinfecting liquid.

4.   A method according to any one of claims 1-3, wherein said metal-EDTA complex is an EDTA Fe • Na complex.

5.   A method according to any of claims 1 to 4, wherein said disinfecting liquid is accommodated in a predetermined container such that the disinfecting liquid in the container has a depth of 3-10 mm, said object is immersed in the disinfecting liquid, and the disinfecting liquid is irradiated with said visible light at least in a direction of the depth of the disinfecting liquid.

6.   A method according to claim 5, wherein said light has a wavelength of 390-600 nm.

7.   A method according to any one of claims 1-6, wherein said disinfecting liquid has an osmotic pressure of 250-350 mOsm.

8.   A method according to any one of claims 1-7, wherein said disinfecting liquid further includes a surfactant.

9.   A method according to any one of claims 1-8, wherein said object is a contact lens.

**Patentansprüche**

1.   Verfahren zum Desinfizieren eines Gegenstands durch das Eintauchen desselben in eine Desinfektionsflüssigkeit, **dadurch gekennzeichnet, dass:**

die Desinfektionsflüssigkeit ein Wasserstoffperoxid und einen Metall-EDTA-Komplex in einem wässrigen Medium umfasst, wobei die Konzentration des Wasserstoffperoxids in der Desinfektionsflüssigkeit im Bereich von 1.000 ppm oder weniger liegt, wobei die Desinfektionsflüssigkeit, in die der Gegenstand eingetaucht wird, weiters mit sichtbarem Licht von einer Leuchtdiode bestrahlt wird.

2. Verfahren nach Anspruch 1, worin die Konzentration des Wasserstoffperoxids in der Desinfektionsflüssigkeit im Bereich von 10 bis 500 ppm liegt.

3. Verfahren nach Anspruch 1 oder 2, worin die Konzentration des Metall-EDTA-Komplexes in der Desinfektionsflüssigkeit im Bereich von 10 bis 1.200 ppm liegt.

4. Verfahren nach einem der Ansprüche 1 bis 3, worin der Metall-EDTA-Komplex ein EDTA-Fe Na-Komptex ist.

5. Verfahren nach einem der Ansprüche 1 bis 4, worin die Desinfektionsflüssigkeit in einem vorbestimmten Behälter so aufgenommen ist, dass die Desinfektionsflüssigkeit in dem Behälter einen Stand von 3 bis 10 mm aufweist, der Gegenstand in die Desinfektionsflüssigkeit eingetaucht wird und die Desinfektionsflüssigkeit mit sichtbarem Licht zumindest in einer Richtung des Desinfektionsflüssigkeitsstands bestrahlt wird.

6. Verfahren nach Anspruch 5, worin das Licht eine Wellenlänge von 390 bis 600 nm aufweist.

7. Verfahren nach einem der Ansprüche 1 bis 6, worin die Desinfektionsflüssigkeit einen osmotischen Druck von 250 bis 350 mOsm aufweist.

8. Verfahren nach einem der Ansprüche 1 bis 7, worin die Desinfektionsflüssigkeit weiters ein Tensid umfasst.

9. Verfahren nach einem der Ansprüche 1 bis 8, worin der Gegenstand eine Kontaktlinse ist.

## Revendications

1. Méthode de désinfection d'un objet en étant immergé dans un liquide de désinfection, **caractérisé en ce que:**

ledit liquide de désinfection contient, dans un milieu aqueux, un peroxyde d'hydrogène et un complexe métallique de l'EDTA, où une concentration dudit peroxyde d'hydrogène est dans une plage de 1000 ppm ou moins dans le liquide de désinfection, où ledit liquide de désinfection, dans lequel ledit objet est immergé, est en outre irradié avec une lumière visible, d'une diode émettrice de lumière.

2. Méthode selon la revendication 1, dans laquelle une concentration dudit peroxyde d'hydrogène est dans une plage de 10-500 ppm dans ledit liquide de désinfection.

3. Méthode selon la revendication 1 ou 2, dans laquelle une concentration dudit complexe métallique de l'EDTA est dans une plage de 10-1200 ppm dans ledit liquide de désinfection.

4. Méthode selon l'une quelconque des revendications 1 à 3, dans laquelle ledit complexe métallique de l'EDTA est un complexe Fe · Na de l'EDTA.

5. Méthode selon l'une quelconque des revendications 1 à 4, dans laquelle ledit liquide de désinfection est renfermé dans un contenant prédéterminé de sorte que le liquide de désinfection dans le contenant a une profondeur de 3-10 mm, ledit objet est immergé dans le liquide de désinfection, et le liquide de désinfection est irradié avec ladite lumière visible au moins dans une direction de la profondeur du liquide de désinfection.

6. Méthode selon la revendication 5, dans laquelle ladite lumière a une longueur d'onde de 390-600 nm.

7. Méthode selon l'une quelconque des revendications 1 à 6, dans laquelle ledit liquide de désinfection possède une pression osmotique de 250-350 mOsm.

8. Méthode selon l'une quelconque des revendications 1 à 7, dans laquelle ledit liquide de désinfection comporte en outre un tensio-actif.

9. Méthode selon l'une quelconque des revendications 1 à 8, dans laquelle ledit objet est une lentille de contact.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 52109953 A
- JP 62153217 A
- JP 63059960 A
- JP 2003135572 A
- EP 1095664 A